(19) Europäisches Patentamt

European Patent Office

Office européen des brevets

(11) Veröffentlichungsnummer: **0 273 258 B1**

(12) **EUROPÄISCHE PATENTSCHRIFT**

(45) Veröffentlichungstag der Patentschrift: **21.11.91**

(51) Int. Cl.⁵: **A61B 5/00**, G01N 33/49

(21) Anmeldenummer: **87118239.0**

(22) Anmeldetag: **09.12.87**

(54) Anordnung zur Untersuchung eines flüssigen Mediums und Verfahren zum Betrieb der Anordnung.

(30) Priorität: **22.12.86 DE 3643980**

(43) Veröffentlichungstag der Anmeldung:
**06.07.88 Patentblatt 88/27**

(45) Bekanntmachung des Hinweises auf die
Patenterteilung:
**21.11.91 Patentblatt 91/47**

(84) Benannte Vertragsstaaten:
**BE CH DE FR GB IT LI LU NL SE**

(56) Entgegenhaltungen:
**EP-A- 0 036 171          EP-A- 0 120 108
EP-A- 0 155 725          DE-A- 3 040 168
US-A- 4 221 567          US-A- 4 535 786**

(73) Patentinhaber: **SIEMENS AKTIENGESELL-
SCHAFT
Wittelsbacherplatz 2
W-8000 München 2(DE)**

(72) Erfinder: **Gumbrecht, Walter, Dr. Dipl.-Chem.
Am Europakanal 2
W-8520 Erlangen(DE)**
Erfinder: **Schelter, Wolfgang, Dr. Dipl.-Phys.
Esperstrasse 3
W-8525 Uttenreuth(DE)**
Erfinder: **Montag, Bernhard
Nordring 22
W-8550 Forchheim(DE)**
Erfinder: **Höbel, Peter, Dipl.-Ing. (FH)
Am Eichengarten 8
W-8520 Buckenhof(DE)**

## Beschreibung

Die Erfindung bezieht sich auf eine Anordnung zur Untersuchung eines flüssigen Mediums, insbesondere zur Blutuntersuchung, mit einem Meßsensor, der sowohl mit dem Meßmedium als auch mit einem Referenzsensor sowie einer Pumpe und einem flüssigen Eichmedium verbunden ist, und mit einer Meßeinrichtung zur Differenzmessung zwischen dem Meßsensor und dem Referenzsensor.

Mit Meßanordnungen zur elektrochemischen Bestimmung von Blutparametern ist eine simultane Patientenüberwachung möglich. Die Konzentrationen wichtiger Blut-Elektrolyten, wie beispielsweise von Kalzium $Ca^{2+}$, Kalium $K^+$ und Natrium $Na^+$, oder auch von Blutgasen, beispielsweise Kohlendioxid oder Sauerstoff, oder auch der pH-Wert sowie deren zeitliche Veränderung im Körper des Patienten werden gemessen. Kalzium steuert im Körper verschiedene biologische Prozesse, beispielsweise die Muskelkontraktion und die Freisetzung von Hormonen. Die klinische Ermittlung und Einstellung solcher Konzentrationen kann deshalb von großer Bedeutung sein. Ferner können Biomoleküle, beispielsweise Glucose, gemessen werden.

Als Meßsensor ist ein ionensensitiver Feldeffekt-Transistor geeignet, der auf einem Silizium-Chip integriert ist. Als Steuerelektrode dient eine Membran, die eine Grenzfläche zum Meßmedium bildet. Gemessen wird die Potentialveränderung durch Verschiebung von Ionen an der Grenzfläche. Dieser unter der Bezeichnung ISFET oder ChemFET bekannten Meßsonde mit direkter Verbindung zum flüssigen Medium wird abwechselnd eine Eichlösung oder das Blut zugeführt. Ein Ventil gibt entweder die Blutströmung oder die Eichlösung frei, die über einen Wärmetauscher strömt und dadurch das zugeführte Blut auf Raumtemperatur abkühlt. Als Referenz ist eine elektrochemische Bezugselektrode vorgesehen, die im wesentlichen aus einer Metallelektrode besteht, die mit einem schwerlöslichen Salz dieses Metalls überzogen ist, in eine Elektrolytlösung eintaucht und mit einem Diaphragma verschlossen ist. Sie ist in der Strömungsrichtung des zugeführten Blutes hinter dem ChemFET angeordnet und kann gegebenenfalls noch über einen Brückenelektrolyten sowie ein weiteres Diaphragma mit der Meßlösung verbunden sein. Eine derartige Meßanordnung ermöglicht zwar eine online-Messung mit einer exvivo-Anordnung des Sensors, während der Eichung stockt jedoch die Blutströmung in der Zuführungsleitung, so daß eine Heparinisierung erforderlich ist. Außerdem ist der gesamte Aufbau verhältnismäßig kompliziert. (Med. & Biol. Engineering & Computing, Juli 1985, Seiten 329 bis 338).

In einer weiteren bekannten Ausführungsform einer Meßanordnung zur Blutanalyse ist ein Chem-FET als Meßsensor vorgesehen, der am Ende eines Katheters an dessen Innenwand angeordnet ist. Dieser Meßsensor ist über eine Schlauchleitung mit einer elektrochemischen Bezugselektrode sowie einer umsteuerbaren Pumpe an einem Behälter für ein Eichmedium angeschlossen. Die Bezugselektrode ist über eine weitere umsteuerbare Pumpe an eine Infusionslösung oder ein Spülmedium angeschlossen. Durch die Pumpen in Verbindung mit Ventilen werden dem Meßsensor abwechselnd Blut oder das Eichmedium oder die Infusionslösung zugeführt. In dieser Ausführungsform müssen somit sowohl das Eichmedium als auch das Spülmedium zur Injektion in den Blutkreislauf des Patienten geeignet sein. Störpotentiale können eine Polarisation der Bezugselektrode bewirken und damit das Meßergebnis verfälschen (deutsches Patent 30 38 883).

Es ist ferner bekannt, daß in einer Anordnung zur Blutuntersuchung als Meßsensor und als Referenzsensor jeweils ein ionensensitiver Feldeffekttransistor vorgesehen sein kann, zwischen denen ein Lösungskontakt angeordnet ist, der auf Nullpotential des Meßsystems liegt. Die Meßstrecke mit den Sensoren ist in einem Katheter angeordnet. Der Meßsensor ist an der Außenseite des Katheters im Blutstrom angeordnet und bleibt in der Blutbahn. In dieser Anordnung kann der Sensor durch Anliegen an der Gefäßwand zugedeckt werden und damit in seiner Funktion beeinträchtigt werden. Ferner kann der Meßsensor nicht freigespült werden und eine Abstoßungsreaktion des Körpers nicht verhindert werden (E-OS 0 155 725).

Mindestens ein Meßsensor und ein Referenzsensor können mit dem Meßkanal auch eine gemeinsame Baueinheit bilden. Als Sensoren dienen ionenselektive Elektroden in Miniaturausführung. Der an einem Ende über einen Katheter mit dem Blutstrom verbundene Meßkanal ist am anderen Ende über eine umsteuerbare Pumpe mit einer Eichlösung verbunden. Zur Eichung wird der Meßkanal zunächst mit der Infusionslösung gefüllt. Anschließend wird Blut so weit in den Meßkanal gezogen, daß sich die Meßelektrode im Blut befindet und dann eine Differenzmessung durchgeführt. Das zur Messung angesaugte Blut wird anschließend in den Blutstrom zurückgeführt (US-Pat. 4 535 786).

Bei dieser bekannten Anordnung wird somit Meßlösung oder auch Eichlösung, die mit wenigstens einem der Sensoren in Berührung gekommen sind, dem Patienten wieder zugeführt. Die Membranen der Sensoren müssen deshalb sterilisiert sein. Ein Austreten toxischer Stoffe aus der Membran oder auch ein Ablösen der Membran kann zu gesundheitlichen Schäden des Patienten führen.

Der Erfindung liegt nun die Aufgabe zugrunde, diese bekannte Ausführungsformen von Meßanordnungen zu vereinfachen und zu verbessern, insbesondere soll das Meßmedium nicht zum Entnahmeort zurückgeführt werden und am Entnahmeort soll außer dem Meßmedium lediglich die Infusionslösung, die nicht mit einem Sensor in Berührung gekommen ist, erscheinen, damit aus den Membranen gelöste Stoffe nicht zum Entnahmeort gelangen und Toxizitäts- oder Sterilitätsprobleme verursachen können.

Es ist ferner bekannt, daß zur Bestimmung von Gasen, Elektrolyten oder Zucker im Blut ein doppellumiger Katheter verwendet werden kann, dessen ein Lumen über eine Elektrodenkammer und eine getrennte Eichkammer an eine umsteuerbare Pumpe angeschlossen ist, die mit dem anderen Lumen direkt verbunden ist. Im Katheter befindet sich zwischen den beiden Lumen eine Öffnung. Die Elektrodenkammer enthält eine $CO_2$- oder pH-Elektrode, eine Sauerstoff-Elektrode und eine Referenz-Elektrode. Eine Trägerlösung wird in einem geschlossenen Kreislauf umgepumpt und nimmt durch eine gasdurchlässige Wandung im Katheder Gase aus dem Blut auf (US-Patent 4 221 567).

Bei einer Glucose-Bestimmung des Blutes ist zur Mischung des entnommenen Blutes mit Heparinlösung auch schon eine Doppellumen-Nadel verwendet worden. Diese Doppelkanüle besteht aus einem dünnen Kunststoffrohr, das zur Abführung des heparinisierten Blutstromes ein etwas kürzeres Innenrohr enthält, dessen Öffnung gegenüber der Mündung des Außenrohres etwas zurückgesetzt ist (Ann. N.Y. Acad. Sc., Band 87 (1960), Seiten 658 bis 668).

Die Erfindung macht Gebrauch von dieser bekannten Einrichtung und sie besteht in den kennzeichnenden Merkmalen des Anspruchs 1. In dieser Ausführungsform der Anordnung, bei der die Infusionslösung der Meßanordnung über den Katheter zugeführt wird, ist weder eine elektrochemische Bezugselektrode mit einem für Verschmutzung anfälligen Diaphragma noch eine besondere Brückenelektrolytlösung erforderlich. Alle Sensoren der Anordnung werden im zeitlichen Mittel mit denselben Lösungen beaufschlagt, es ist somit eine ideale Driftkompensation gewährleistet. Obwohl in dieser Anordnung die beiden Sensoren jeweils abwechselnd sowohl als Meßsensor als auch als Referenzsensor dienen, wird in der folgenden Beschreibung die Bezeichnung Meßsensor und Referenzsensor beibehalten.

Als Meß- und Referenzsensor können vorzugsweise ChemFETs vorgesehen sein, von denen die einander zugeordneten Meß- bzw. Referenzsensoren mit gleichen Membranen versehen sind. Der Meß-kanal zwischen den beiden ChemFETs kann mit dem Deckschip vorzugsweise als Wärmetauscher ausgebildet sein. Beispielsweise kann durch eine Mäanderform des Durchflußkanals, der vorzugsweise als Rille im Deck-Chip ausgebildet sein kann, eine ausreichende Wärmeübertragung zur Meßlösung sichergestellt werden.

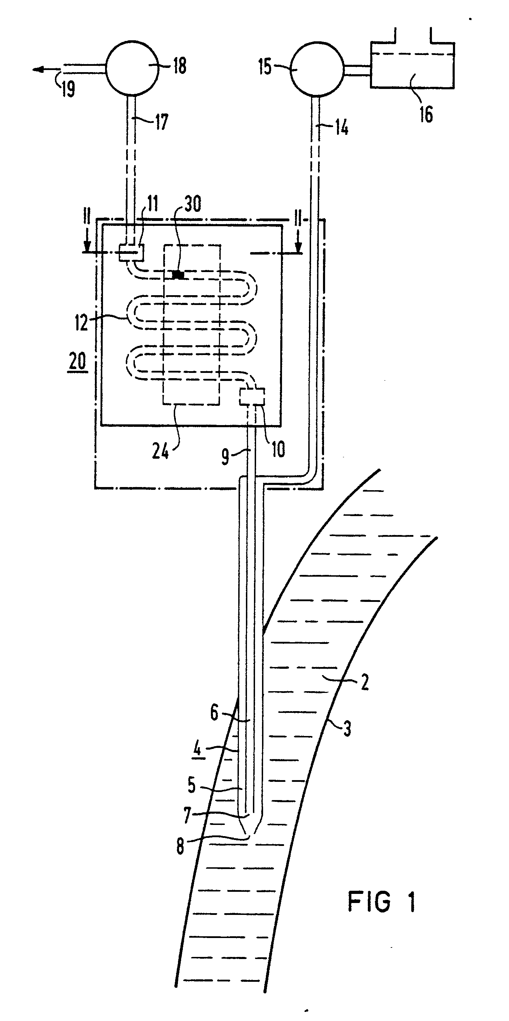

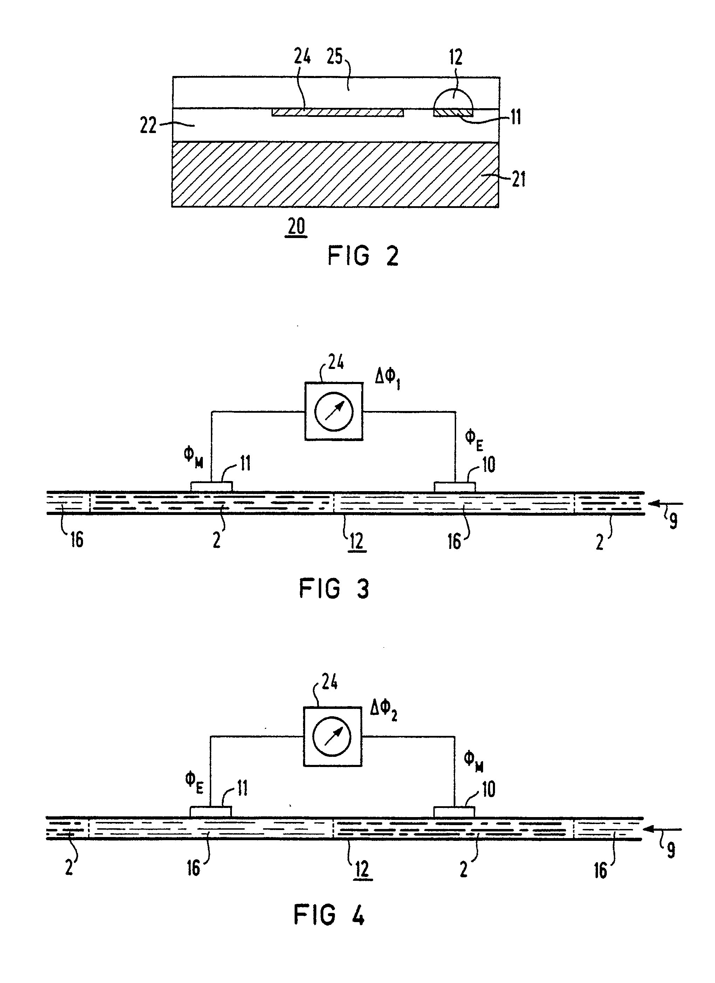

Zur weiteren Erläuterung der Erfindung wird auf die Zeichnung Bezug genommen, in der ein Ausführungsbeispiel einer Anordnung zur Untersuchung eines flüssigen Meßmediums gemäß der Erfindung schematisch veranschaulicht ist. Figur 1 zeigt ein Übersichtsbild der Meßanordnung als Draufsicht. In Figur 2 ist ein Schnitt durch einen Teil der Figur 1 dargestellt. Die Figuren 3 und 4 dienen zur Erläuterung eines vorteilhaften Betriebsverfahrens für die Anordnung.

In der Anordnung gemäß Figur 1 zur Untersuchung von Blut als flüssiges Meßmedium 2, das durch die Ader 3 eines in der Figur nicht dargestellten Patienten strömt, ist ein zweilumiger Katheter 4 mit beispielsweise konzentrisch angeordneten Lumen in den Blutstrom eingeführt. Das äußere Lumen 5, dessen Mündung 8 in den Blutstrom reicht, ist über einen Infusionsschlauch 14 sowie eine Vorrichtung zur Umkehr der Strömungsrichtung, insbesondere eine umsteuerbare Pumpe 15, an einen Behälter mit einer Infusionslösung 16 angeschlossen. Das innere Lumen 6, dessen Mündung 7 sich innerhalb des äußeren Lumens 5 befindet, ist über einen Infusionsschlauch 9 und einen Meßkanal 12 sowie einen Infusionsschlauch 17 mit einer Vorrichtung 18 für eine vorbestimmte Ausflußgeschwindigkeit am Ausgang 19 verbunden.

Als Vorrichtung 18 kann vorzugsweise eine Schlauchpumpe verwendet werden, die einen konstanten Meßstrom gewährleistet. Ferner ist beispielsweise auch ein Drosselventil geeignet. Der Meßkanal 12 verbindet zwei Sensoren, insbesondere ChemFETs, die in der folgenden Beschreibung als Meßsensor 10 und Referenzsensor 11 bezeichnet werden, obwohl sie in dieser Anordnung jeweils abwechselnd sowohl als Meßsensor als auch als Referenzsensor dienen.

Die Meß- und Referenzsensoren 10 bzw. 11 können vorzugsweise auf einem gemeinsamen Chip 22 integriert sein. Ihnen ist eine gemeinsame Auswerteelektronik 24 und gegebenenfalls noch ein Temperatursensor zugeordnet, die vorzugsweise ebenfalls mit dem Chip 22 eine Baueinheit bilden, insbesondere auf diesem Chip integriert sein können. Damit erhält man eine sehr kompakte Bauform des Sensorblockes 20, dessen gesamte Ausdehnung A etwa 10 mm nicht wesentlich überschreitet und insbesondere weniger als 10 mm betragen kann und der vorzugsweise mit dem Katheter 4 eine gemeinsame Baueinheit bilden kann. Der Meßanordnung ist noch ein elektrischer Lösungskontakt 30 zugeordnet, der die Infusionslösung, die zugleich als Referenz- und Spüllösung dient, kontaktiert. Dieser Lösungskontakt 30 kann im Strömungskanal 9 oder dem Strömungskanal 17 vorzugsweise im Meßkanal 12 zwischen den beiden ChemFETs 10 und 11 insbesondere in der Nähe des Referenzsensors 11 angeordnet sein. Der Lösungskontakt 30 stellt einen elektrischen Kontakt her zwischen dem Meßmedium 2 bzw. der Infusionslösung 16 und dem elektrischen Bezugsnullpunkt der Auswerteelektronik 24.

Sowohl das gezogene Meßmedium 2 als auch die gezogene Infusionslösung 16 verlassen die Anordnung am Ausgang 19.

In einer besonders einfachen Ausführungsform der Anordnung gemäß Figur 1 kann zur Umsteuerung der Strömungsrichtung in der Katheteröffnung 8 beispielsweise auch ein Quetschventil vorgesehen sein.

Gemäß Figur 2 kann der Sensorblock 20 eine Grundplatte 21 enthalten, die mit dem Chip 22 versehen ist, der vorzugsweise aus Silizium bestehen und auch noch die Auswerteelektronik 24 enthalten kann. Der Sensorblock 20 ist mit einem Deck-Chip 25 abgedeckt. Im Chip 22 ist auch der Meßsensor 10 und der Referenzsensor 11 integriert. Der Strömungskanal 12 kann vorzugsweise als Rille, insbesondere mäanderförmige Rille, in den Deckchip 25 eingearbeitet sein.

In einer besonderen Ausführungsform kann der Deck-Chip 25 noch eine in der Figur nicht dargestellte Heizeinrichtung enthalten, die zur Thermostatisierung des Meßmediums 2 und der Infusionslösung 16 im Meßkanal 12 dienen kann. Zu diesem Zweck kann beispielsweise die Grundplatte 21, vorzugsweise der Deck-Chip 25, mit einer in der Figur nicht dargestellten Halbleiter-Widerstandsheizung oder auch einer Metall-Widerstandsheizung versehen sein. In dieser Ausführungsform wird die Meßanordnung auf die Körpertemperatur des Patienten aufgeheizt, so daß eine rechnerische pH-Wert-Korrektur überflüssig wird, die beispielsweise beim Abkühlen von Körpertemperatur auf Raumtemperatur eine Korrektur erforderlich machen würde, die in der Größenordnung des Meßwertes liegen kann.

Zur Inbetriebnahme der Meßanordnung wird zunächst die Infusionslösung 16, vorzugsweise eine physiologische Elektrolytlösung, insbesondere eine Ringer-Lösung, mit Hilfe der Pumpe 15 durch den Infusionsschlauch 14 in das äußere Lumen 5 des Katheters 4 bis in den Blutkreislauf des Patienten gepumpt. Gleichzeitig wird durch die Vorrichtung 18 über die Öffnung 7 des Lumens 6 ein Teil dieser Infusionslösung über den Meßsensor 10 und den Referenzsensor 11 gezogen.

Mit der Umkehrung der Strömung an der Mündung 8, beispielsweise durch Umsteuerung der Pumpe 15, wird an der Mündung 7 des Lumens 6 Meßlösung 2 gezogen, die in das Lumen 6 einströmt. Sobald eine vorbestimmte Menge der Meßlösung 2 in das innere Lumen 6 eingeströmt ist, wird die Pumpe 15 wieder umgepolt.

Im Rhythmus der Umsteuerung der Pumpe 15 wird von der Vorrichtung 18 eine Folge von jeweils abwechselnd gleichen Raumteilen des Meßmediums 2 und der Infusionslösung 16 über die Sensoren 10 und 11 gezogen. Die Größe der Raumteile im Meßkanal 12 wird gemäß Figur 3 so gewählt, daß jeweils abwechselnd der eine Sensor, beispielsweise der Referenzsensor 11, an der Meßlösung 2 anliegt, wenn der andere Sensor, beispielsweise der Meßsensor 10, an der Infusionslösung 16 anliegt. Es dient somit jeweils abwechselnd der eine Sensor als Meß- und der andere als Referenzsensor.

Bei einer Messung mit der Auswerteelektronik 24, die in Figur 3 zur Vereinfachung lediglich als Meßinstrument dargestellt ist, erhält man mit einem Meßpotential $\Phi_M$ und einem Eichpotential $\Phi_E$ eine Potentialdifferenz

$$\Delta \Phi_1 = \Phi_E - \Phi_E$$

und mit der folgenden Messung gemäß Figur 4 erhält man eine Potentialdifferenz

$$\Delta \Phi_2 = \Phi_E - \Phi_M = \Phi_1.$$

Im Ausführungsbeispiel gemäß Figur 1 ist ein Katheter dargestellt, bei dem ein Lumen von dem anderen konzentrisch umgeben ist. Es können jedoch beispielsweise auch zwei oder mehrere getrennte Lumen mit einer gemeinsamen Öffnung zum Meßmedium 2 nebeneinander angeordnet sein. Ferner kann das äußere Lumen auch mehrere getrennte weitere Lumen enthalten.

Abweichend von der Darstellung in den Figuren 3 und 4 kann auch jeweils nach einer Messung, beispielsweise gemäß Figur 3, wieder eine Eichung stattfinden. In diesem Falle liegt dann nach jeder Messung die Infusionslösung 16 an den beiden Sensoren an. Bei dieser Betriebsweise ist der Kontakt zwischen Sensor und Meßlösung 2 zeitlich reduziert und somit die Gefahr einer Kontamination, beispielsweise eine Ablagerung von Proteinen auf der Sensor-Membran, entsprechend gering.

Im Ausführungsbeispiel ist die Verwendung der Anordnung zur Untersuchung eines flüssigen Meßmediums zur Blutuntersuchung vorgesehen. Die Anordnung kann jedoch auch auf anderen technischen Sachgebieten vorteilhaft angewendet werden, beispielsweise in der Biotechnologie zur Prozeßkontrolle und Prozeßsteuerung in einem Bioreaktor sowie beispielsweise zur fortlaufenden Registrierung von pH-Werten und der Konzentration schädlicher Ionen in Gewässern.

**Patentansprüche**

1. Anordnung zur Untersuchung eines flüssigen Mediums (2) mit einem Meßkanal (12), der an einen Katheter (4) angeschlossen ist und der mit einem Meßsensor (10) und einem Referenzsensor (11) versehen ist und dem das Meßmedium (2) oder eine Infusionslösung (16) zuführbar ist, und mit einer Meßeinrichtung zur Differenzmessung zwischen dem Meßsensor (10) und dem Referenzsensor (11), **gekennzeichnet** durch folgende Merkmale:

    a) der Katheter (4) enthält mehrere Lumen (5, 6) mit einer gemeinsamen Mündung (8) zum Meßmedium (2)

    b) mindestens ein Lumen (5) ist jeweils an eine Infusionslösung (16) angeschlossen, dessen Strömungsrichtung an der Mündung (8) des Katheters (4) umkehrbar ist

    c) ein anderes Lumen (6) ist an den Meßkanal (12) angeschlossen, der mit einer Vorrichtung (18) für eine vorbestimmte Ausströmungsgeschwindigkeit versehen ist

    d) der Meßkanal (12) verbindet wenigstens einen Meßsensor (10) mit wenigstens einem Referenzsensor (11)

    e) die Größe des Meßkanals (12) ist so gewählt, daß jeweils abwechselnd der eine Sensor (z.B. 11) an der Infusionslösung (16) und gleichzeitig der andere Sensor (z.B. 10) an der Meßlösung (2) anliegt.

2. Anordnung nach Anspruch 1, **dadurch gekennzeichnet,** daß als Meßsensor (10) und als Referenzsensor (11) jeweils ein ionenselektiver Feldeffekttransistor (Chem-FET) vorgesehen ist.

3. Anordnung nach Anspruch 2, **dadurch gekennzeichnet,** daß Feldeffekttransistoren mit der gleichen Membran vorgesehen sind.

4. Anordnung nach einem der Ansprüche 1 bis 3, **dadurch gekennzeichnet,** daß der Meßsensor (10) und der Referenzsensor (11) mit dem Meßkanal (12) einen gemeinsamen Sensorblock (20) bilden.

5. Anordnung nach einem der Ansprüche 1 bis 3, **gekennzeichnet** durch die Integration des Meßsensors (10) und des Referenzsensors (11) auf einem gemeinsamen Chip (22).

6. Anordnung nach Anspruch 5, **dadurch gekennzeichnet,** daß auch eine Auswerteelektronik (24) auf dem Chip (22) integriert ist.

7. Anordnung nach Anspruch 5 oder 6, **gekennzeichnet** durch die Ausführung des Meßkanals (12) als Rille in einem Deck-Chip (25).

8. Anordnung nach Anspruch 5, **dadurch gekennzeichnet,** daß der Sensorblock (20) mit einer Heizung versehen ist.

9. Anordnung nach einem der Ansprüche 1 bis 8 mit einem Referenzsensor, dem ein Lösungskontakt zugeordnet ist, **dadurch gekennzeichnet,** daß der Lösungskontakt (30) im Meßkanal (12) zwischen dem Meßsensor (10) und dem Referenzsensor (11) angeordnet ist.

10. Anordnung nach einem der Ansprüche 1 bis 3, **dadurch gekennzeichnet,** daß zwischen einem Behälter für die Infusionslösung (16) und dem Katheter (4) eine umsteuerbare Pumpe (15) vorgesehen ist.

11. Anordnung nach einem der Ansprüche 1 bis 9, **dadurch gekennzeichnet,** daß zwischen einem Behälter für die Infusionslösung (16) und dem Katheter (4) ein Quetschventil vorgesehen ist.

12. Anordnung nach einem der Ansprüche 1 bis 11, **dadurch gekennzeichnet,** daß ein Katheter mit weiteren Lumen vorgesehen ist, die jeweils an eine weitere Infusionslösung angeschlossen sind.

13. Anordnung nach Anspruch 12, **dadurch gekennzeichnet,** daß wenigstens eines der weiteren Lumen zur Blutdruckmessung vorgesehen ist.

14. Anordnung nach einem der Ansprüche 1 bis 13 mit jeweils mehreren Meßsensoren und Referenzsensoren, **dadurch gekennzeichnet,** daß den Meßsensoren jeweils ein Referenzsensor mit der gleichen Membran zugeordnet ist.

15. Anordnung nach einem der Ansprüche 1 bis 11 mit wenigstens mehreren Meßsensoren, **dadurch gekennzeichnet,** daß mehreren Meßsensoren ein gemeinsamer Referenzsensor zugeordnet ist.

16. Verfahren zum Betrieb der Anordnung nach einem der Ansprüche 1 bis 15, **dadurch gekennzeichnet,** daß eine vorbestimmte, wenigstens annähernd konstante Strömungsgeschwindigkeit im Meßkanal (12) eingestellt wird und daß durch periodische Umsteuerung der Strömung der Infusionslösung (16) dem Meßsensor (10) und dem Referenzsensor (11) jeweils abwechselnd das Meßmedium (2) und die Infusionslösung (16) derart zugeführt wird, daß der Meßsensor (10) an dem Meßmedium (2) liegt, wenn der Referenzsensor (11) an der Infusionslösung (16) liegt und umgekehrt.

## Claims

1. Arrangement for the examination of a liquid medium (2) having a measuring canal (12) which is attached to a catheter (4) and which is provided with a measuring sensor (10) and a reference sensor (11) and to which the measuring medium (2) or an infusion solution (16) can be supplied, and having a measuring device for the differential measurement between the measuring sensor (10) and the reference sensor (11), characterized by the following features:

   a) the catheter (4) contains several lumens (5, 6) with a common aperture (8) to the medium to be measured (2)

   b) at least one lumen (5) is in each case attached to an infusion solution (16), the flow direction of which is reversible at the aperture (8) of the catheter (4)

   c) a different lumen (6) is attached to the measuring canal (12) which is provided with a device (18) which predetermines the speed of outflow

   d) the measuring canal (12) connects at least one measuring sensor (10) with at least one reference sensor (11)

   e) the size of the measuring canal (12) is chosen such that in each case alternately the one sensor (e.g. 11) abuts the infusion solution (16) and at the same time the other sensor (e.g. 10) abuts the solution (2) to be measured.

2. Arrangement according to claim 1, characterized in that an ion-selective field effect transistor (Chem-FET) is provided in each case as measuring sensor (10) and as reference sensor (11).

3. Arrangement according to claim 2, characterized in that field effect transistors with the same membrane are provided.

4. Arrangement according to one of claims 1 to 3, characterized in that the measuring sensor (10) and the reference sensor (11) with the measuring canal (12) form a common sensor block (20).

5. Arrangement according to one of claims 1 to 3, characterized by the integration of the measuring sensor (10) and the reference sensor (11) on a common chip (22).

6. Arrangement according to claim 5, characterized in that evaluation electronics (24) are also integrated on the chip (22).

7. Arrangement according to claim 5 or 6, characterized by the construction of the measuring canal (12) as groove in a cover-chip (25).

8. Arrangement according to claim 5, characterized in that the sensor block (20) is provided with a heating means.

9. Arrangement according to one of claims 1 to 8, with a reference sensor with which there is associated a solution contact, characterized in that the solution contact (30) in the measuring canal (12) is arranged between the measuring sensor (10) and the reference sensor (11).

10. Arrangement according to one of claims 1 to 3, characterized in that a reversible pump (15) is provided between a container for the infusion solution (16) and the catheter (4).

11. Arrangement according to one of claims 1 to 9, characterized in that a pinching valve is provided between a container for the infusion solution (16) and the catheter (4).

12. Arrangement according to one of claims 1 to 11, characterized in that a catheter with further lumens is provided, which in each case are attached to a further infusion solution.

13. Arrangement according to claim 12, characterized in that at least one of the further lumens is provided for the measurement of blood pressure.

14. Arrangement according to one of claims 1 to 13 with, in each case, several measuring sensors and reference sensors, characterized in that a reference sensor with the same membrane is associated in each case with the measuring sensors.

15. Arrangement according to one of claims 1 to 11 with at least several measuring sensors, characterized in that a common reference sensor is associated with several measuring sensors.

16. Method for the operation of the arrangement according to one of claims 1 to 15, characterized in that a predetermined flow speed, at least approximately constant, is set in the measuring canal (12) and in that, through periodical reversal of the flow of the infusion solution (16), the medium (2) to be measured and the infusion solution (16) are supplied to the measuring sensor (10) and the reference sensor (11) in each case alternately in such a way that the measuring sensor (10) abuts the medium (2) to be measured when the reference sensor (11) abuts the infusion solution (16) and vice versa.

**Revendications**

1. Dispositif d'analyse d'un milieu (2) liquide, comprenant un canal de mesure (12), qui est raccordé à un cathéter (4) et qui est muni d'un capteur de mesure (10) et d'un capteur de référence (11) et auquel peut être amené le milieu à mesurer (2) ou une solution de perfusion (16), et un dispositif de mesure destiné à effectuer une mesure différentielle entre le capteur de mesure (10) et le capteur de référence (11), remarquable par les caractéristiques suivantes :
   a) le cathéter (4) comporte plusieurs conduits (5,6) ayant une embouchure (8) commune dans le milieu à mesurer (2),
   b) au moins un conduit (5) est raccordé à une solution de perfusion (16), dont le sens d'écoulement peut être inversé à l'embouchure (8) du cathéter (4),
   c) un autre conduit (6) est raccordé au canal de mesure (12), qui est muni d'un dispositif (18) pour obtenir une vitesse de sortie de l'écoulement déterminée à l'avance,
   d) le canal de mesure (12) relie au moins un capteur de mesure (10) à au moins un capteur de référence (11),
   e) la dimension du canal de mesure est telle qu'alternativement l'un des capteurs (par exemple 11) s'applique à la solution de perfusion (16) et en même temps l'autre capteur (par exemple 10) à la solution à mesurer (2).

2. Dispositif selon la revendication 1, caractérisé en ce que comme capteur de mesure (10) et comme capteur de référence (11), sont prévus respectivement un transistor à effet de champ sélectif aux ions (Chem-FET).

3. Dispositif selon la revendication 2, caractérisé en ce qu'il est prévu des transistors à effet de champ ayant la même membrane.

4. Dispositif selon l'une des revendications 1 à 3, caractérisé en ce que le capteur de mesure (10) et le capteur de référence (11) forment, avec le canal de mesure (12), un bloc-capteur (20) commun.

5. Dispositif selon l'une des revendications 1 à 3, caractérisé par l'intégration du capteur de mesure (10) et du capteur de référence (11) sur une puce (22) commune.

6. Dispositif selon la revendication 5, caractérisé en ce qu'une électronique d'exploitation (24) est également intégrée sur la puce (22).

7. Dispositif selon la revendication 5 ou 6, caractérisé en ce que le canal de mesure (12) est constitué sous la forme d'une rainure dans une puce de couverture (25).

8. Dispositif selon la revendication 5, caractérisé en ce que le bloc-capteur (20) est muni d'un dispositif de chauffage.

9. Dispositif selon l'une des revendications 1 à 8 comprenant un capteur de référence auquel est associé un contact pour une solution, caractérisé en ce que le contact (30) pour une solution est disposé dans le canal de mesure (12) entre le capteur de mesure (10) et le capteur de référence (11).

10. Dispositif suivant l'une des revendications 1 à 3, caractérisé en ce qu'il est prévu une pompe (15) à débit réversible entre une cuve pour la solution de perfusion (16) et le cathéter (4).

11. Dispositif selon l'une des revendications 1 à 9, caractérisé en ce qu'il est prévu une soupape à pincement entre une cuve pour la solution de perfusion (16) et le cathéter (4).

12. Dispositif selon l'une des revendications 1 à 11, caractérisé en ce qu'il est prévu un cathéter ayant d'autres conduits qui sont raccordés respectivement à une autre solution de perfusion.

13. Dispositif selon la revendication 12, caractérisé en ce qu'au moins l'un des autres conduits est prévu pour mesurer la pression artérielle.

14. Dispositif selon l'une des revendications 1 à 3, comprenant plusieurs capteurs de mesure et plusieurs capteurs de référence, caractérisé en ce qu'aux capteurs de mesure est respectivement associé un capteur de référence ayant la même membrane.

15. Dispositif selon l'une des revendications 1 à 11 ayant au moins plusieurs capteurs de mesure, caractérisé en ce qu'un capteur de référence commun est associé à plusieurs capteurs de mesure.

16. Procédé pour la mise en oeuvre du dispositif selon l'une des revendications 1 à 15, caractérisé en ce qu'il consiste à établir une vitesse d'écoulement au moins approximativement constante et déterminée à l'avance dans le canal de mesure (12), et à envoyer, en inversant périodiquement le courant de la solution de perfusion (16), alternativement le milieu à mesurer (2) et la solution de perfusion (16) au capteur de mesure (10) et au capteur de référence (11), et à appliquer le capteur de mesure (10) au milieu à mesurer (2) quand le capteur de référence (11) est appliqué à la solution de perfusion (16), et inversement.

FIG 1

FIG 2

FIG 3

FIG 4